# EUROPEAN PATENT SPECIFICATION

(11) **EP 4 278 978 B1**
(45) Date of publication and mention of the grant of the patent: **18.02.2026**
(21) Application number: 22173675.4
(22) Date of filing: 17.05.2022
(51) Int. Cl.: A61B 7/00, A61B 5/00

(54) **BRUXISM DETECTION DEVICE AND METHOD OF CONFIGURING SUCH DEVICE**
BRUXISMUS-ERKENNUNGSGERÄT UND VERFAHREN ZUM KONFIGURIEREN EINES SOLCHEN GERÄTS
DISPOSITIF DE DÉTECTION DE BRUXISME ET PROCEDE DE CONFIGURATION D'UN TEL DISPOSITIF

(43) Date of publication of application: 22.11.2023
(73) Proprietor: Universität Basel, 4001 Basel (CH)
(72) Inventor: Nahhas, Mohammad Khair, 4123 Allschwil (CH); Rauter, Georg, 5074 Eiken (CH); Wilhelm, Elisabeth, 9743NB Groningen (NL)
(74) Representative: Latscha Schöllhorn Partner AG

(56) References cited:
- WO-A1-2019/079909
- WO-A1-98/51218
- US-A1- 2019 343 452

## Description

### Technical Field

The present invention relates to a bruxism detection device according to the preamble of independent claim 1 and more particularly to method of configuring a bruxism detection device. The invention may particularly allow to detect acoustic emissions generated in relation with bruxism and to classify them as to being an acoustic emission related to either tooth grinding or jaw clenching.

Such bruxism detection devices can be used for home monitoring and thereby facilitating monitoring in a real-world setting/environment.

### Background Art

Bruxism being tooth grinding and/or jaw clenching is a parafunctional orofacial behaviour. It is defined as masticatory muscle activities that occur during sleep and/or wakefulness. This parafunctional orofacial behaviour occurs in a moderate or in a severe form of bruxism. Typically, it is harmful in as such as it leads to health problems such as temporomandibular pain, tooth wear, or anterior disc displacement due to the loads introduced on the masticatory muscles, teeth and temporomandibular joints. In order to be able to detect, record, monitor and/or manage bruxism different setups are used. For instance, the gold standard for the diagnosis of sleep bruxism is polysomnography (PSG) with audio and video recordings. Such an approach, however, is very resource-intensive and requires the stay in a sleep laboratory. Personal reports are another approach to detect sleep or awake bruxism but are not very reliable and often fail to detect severe or moderate bruxism. Prior art document US 2019/343452 discloses a bruxism monitoring and prevention system that includes a bruxism detection subsystem that is dimensioned to be positioned at least partly in an ear canal of a patient. The bruxism detection subsystem detects, when the bruxism detection subsystem is positioned at least partly in the ear canal, a change in a size of the ear canal of the patient and, in response, generates a bruxism event detection signal. A bruxism notification subsystem is coupled to the bruxism detection subsystem. The bruxism notification subsystem receives the bruxism event detection signal generated by the bruxism detection subsystem and, in response, generates a tone that is configured to be audible to the patient. In prior art document WO 98/51218, which is considered to be the closest prior art, a system is provided for monitoring an undesired behavioural disorder such as bruxism, jaw clenching, or snoring. A processor correlates the monitored behaviour with the onset of the undesired disorder.

Therefore, a flexible and mobile bruxism identification system that is not limiting the user in its every day/night life and that enables the monitoring of both, sleep and awake bruxism in a reliable way may be beneficial. In particular, there is a need for a device allowing the efficient detection of bruxism caused by both, jaw clenching and tooth grinding.

### Disclosure of the Invention

According to the invention this need is settled by a bruxism detection device as it is defined by the features of independent claim 1, and by a method for configuring a bruxism detection device as it is defined by the features of independent claim 10.

Preferred embodiments are subject of the dependent claims.

In one aspect, the invention, as defined in independent claim 1, relates to a bruxism detection device comprising an earplug body, a sound sensing transducer and a processing unit. The ear plug body is designed to be positioned in an ear canal of a user and the sound sensing transducer is mounted to the earplug body such that the sound sensing transducer is directed towards a tympanic membrane of the user when the earplug body is positioned in the ear canal of the user. The processing unit is connected to the sound sensing transducer such that a sound signal detected by the sound sensing transducer is receivable by the processing unit. The processing unit is configured to evaluate the sound signal received from the sound sensing transducer in order to detect sound associated to tooth grinding and in order to detect sound associated to jaw clenching.

The term "earplug body" in accordance with the invention relates to a means or element which is designed to be positioned in the ear canal of the user or in the direct vicinity of the ear canal. Thereby, the earplug body can be embodied to be conveniently positioned and worn in the ear canal. Depending on the purpose, each ear of a user can be plugged with such earplug body. Thus, advantageously, the bruxism detection device comprises a further earplug body equipped with a further sound sensing transducer.

The earplug body may comprise an attachment structure for the sound sensing transducer and/or a sound generating transducer. Alternatively, the sound sensing transducer and/or the sound generating transducer may be an integral part of the earplug body.

The earplug preferably is designed to occlude the ear canal when being positioned in the ear canal of a user. In order to receive the best quality of sounds stemming from the user's head such as sounds from jaw clenching, tooth grinding, active masticatory or other muscles, temporomandibular joint, blood vessels in the ear and/or pressure changes in the inner ear, it is advantageous to occlude the ear of the user, which can be achieved by the accordingly designed earplug. For achieving occlusion or soundproof closure of the ear canal, it can be plastically or more advantageously elastically deformable.

The term "sound sensing transducer" in accordance with the invention relates to a means which transforms sound waves into an electric or digital signal. The electric or digital signal provided by the sound transducer is referred to as sound signal. The sound waves of the sound signal can be any kind of sound waves including airborne soundwaves and bone conducted soundwaves. In an advantageous embodiment, the sound transducer is a microphone. In embodiments of the invention the sound sensing transducer can be a sensor like, e.g., a voice pick-up sensor.

The processing unit can be integrated in the earplug body. However, advantageously it is a separate entity which is preferably wirelessly connected to the earplug body or, more specifically, to the sound sensing transducer mounted to the earplug body and, eventually, to a sound generating transducer mounted to the earplug body. For wireless communication, the earplug body can comprise an antenna such as a Bluetooth antenna, a wire or a wire plug for transferring electric or digital signals to the processing unit and eventually, from the sound generating transducer mounted to the earplug body.

When being a separate entity, the processing unit can be embodied in a portable or non-portable computing device such as smartphone, a laptop computer, a desktop computer or the like. Such separate unit may also comprise a data storage to record and save the sound signals or other data or information received by the earplug body or, more specifically, to the sound sensing transducer mounted to the earplug body. Moreover, the separate processing unit can be configured to perform real-time sound analysis to record only the recognized labels such as, e.g., no bruxism, jaw clenching, or tooth grinding, in combination with a time signal. This may avoid creating data security/privacy issues because sounds will not be recorded, only the resulting labels and time stamps. So a medical doctor can find out how often and which kind of bruxism-related activities are happening. The labels can also be finer classified such as heavy/moderate/mild grinding or clenching Furthermore, such separate unit may be embodied with an interface to a network such as the Internet for storing or processing the sound signals or data or information received by the earplug body or, more specifically, to the sound sensing transducer mounted to the earplug body.

The device according to the invention benefits from an approach to detect bruxism by the use of acoustic emissions caused by the orofacial behaviour characteristic to bruxism. In particular, it has been found that there may be at least three different but still interlinked mechanisms by which bruxism causes acoustic emissions. These acoustic emissions are detectable in the ear canal of a user which mechanism is used by the device according to the invention. The first mechanism is that sounds induced by, e.g., tooth grinding do travel across the head via bone propagation. The second mechanism is the alternation of the blood flow in the ear of a user as well as the affected muscles by the activation of the different masticatory muscles associated with bruxism, namely masseter, temporalis and pterygoid as well as the middle ear muscle. The third mechanism relates to the activation of the middle ear muscle by, e.g., jaw clenching resulting in the alternation of pressure inside an occluded ear canal due to the deformation of the tympanic membrane, which is acoustically detectable. For the detection of each of these mechanisms the sound sensing transducer is provided which needs to be placed in the ear canal of the user by means of properly setting the earplug body.

When the source of the acoustic emission is tooth grinding, the sound signal, travels across the users head via bone propagation and is detectable by e.g. the sound sensing transducer in the ear canal of the user. These detected sound signals are the sound features associated to tooth grinding. With regard to tooth grinding sound signals generated by the alternation of blood flow in the ear of the user as well as in affected muscles by the activation of the different masticatory muscles associated with bruxism are detectable in the ear canal of the user too. Further the pressure in the occluded ear canal may vary due to the activation of the middle ear muscle while tooth grinding. The sound emission related to the pressure alternation are detectable by the sound sensing transducer.

When the source of the acoustic emission is jaw clenching, sound signals generated by the alternation of blood flow in the ear of the user as well as in affected muscles by the activation of the different masticatory muscles associated with jaw clenching in connection with bruxism are detectable in the ear canal of the user. Further the pressure in the occluded ear canal may alter due to the activation of the middle ear muscle while jaw clenching. The sound emission related to the pressure alternation are detectable by the sound sensing transducer. The sensed sound features (e.g. wavelength, frequency, speed and amplitude) do differ from the sound features sensed in the context of tooth grinding.

The device according to the invention allows for efficiently detecting bruxism of the user. Moreover, the different bruxism behaviour can be separately identified such that a specific treatment can be applied. Like this, success rate of the treatment can be increased. Furthermore, the bruxism detection device can be embodied to be comparably little disturbing in use. Like this, it can be achieved that the device is worn by the user also during daytime and treatment of daytime bruxism is efficiently possible. Thus, the bruxism detection device according to the invention allows less cumbersome and more reliable detection of bruxism caused by both, jaw clenching and tooth grinding.

Preferably, the processing unit of the bruxism detection device is configured to evaluate the sound signal received from the sound sensing transducer by means of a machine learning algorithm. By the processing unit being configured in that manner, efficient associating sensed sound to sound indicative for jaw clenching and/or tooth grinding can be achieved. In particular, such configuration allows for efficiently setting respective reference sounds with no need for exactly defining the single sound features involved in theory. Rather, practically measured sensed sound may be used in an accurate and efficient manner.

Thereby, various machine learning algorithms may be applied. Also, a combination of machine learning algorithms may be used which can increase quality of the reference sounds. Thus, any algorithm or algorithm combination suitable for solving the problem of detecting the sound feature(s) to be associated to tooth grinding or those of jaw clenching from the sound signals sensed by the sound sensing transducer can be used, i.e. bruxism classification. The machine learning algorithm used for determining the sound features associated to tooth grinding can be the same as or different from the machine learning algorithm used for determining the sound features associated to jaw clenching.

Preferably, the processing unit is configured to evaluate the sound signal received from the sound sensing transducer by comparing sound features predefined to be associated to tooth grinding to sound features of the sound signal received from the sound sensing transducer. The term "sound feature" as used herein relates to any characteristic of the sound signal. Such sound features may include data provided or measured by the transducer or information transformed or evaluated from the measured data. Additionally or alternatively, the processing unit preferably is configured to evaluate the sound signal received from the sound sensing transducer by comparing sound features predefined to be associated to jaw clenching to sound features of the sound signal received from the sound sensing transducer. Such comparison allows for an efficient detection of tooth grinding and/or of jaw clenching. The sound features may comprise at least one of wavelength, frequency, speed and amplitude.

Thereby, the sound features of the sound signal received from the sound sensing transducer can be aggregated to a sound pattern. Similarly, the sound features predefined to be associated to tooth grinding and/or to jaw clenching can be aggregated to a tooth grinding reference pattern or a jaw clenching reference pattern. In such embodiments, the sound pattern can be compared to the tooth grinding reference pattern and/or the jaw clenching patter such that an efficient detection of tooth grinding and/or jaw clenching can be achieved.

Preferably, the bruxism detection device comprises a sound generating transducer mounted to the earplug body such that the sound generating transducer is directed towards the tympanic membrane of the user, when the earplug body is positioned in the ear canal of the user.

The term "sound generating transducer" in this connection relates to a means or structure which transforms electric or digital signals to acoustic signals. In an embodiment the sound generating transducer is a loud speaker or an external device as described above.

Such sound generating transducer can be embodied as signaling as described below. In particular, the earplug body may comprise the sound generating transducer to send an alert signal to the user or a practitioner supervising the user via the external control unit.

In a preferred embodiment, the processing unit is connected to the sound generating transducer and the processing unit is configured to control the sound generating transducer to generate a calming sound when sound associated to tooth grinding and/or sound associated to jaw clenching is detected. Like this, the user can be calmed such that the tooth grinding and/or jaw clenching is stopped.

Preferably, the bruxism detection device comprises a signaling unit adapted to generate a signal recognizable by the user, e.g., an alert signal. Such signal may be an acoustic signal, a visual signal, a haptic signal or any combination thereof.

Thereby, the processing unit preferably is configured to activate the signaling unit, when sound associated to tooth grinding is detected. Additionally or alternatively, the processing unit preferably is configured to activate the signaling unit, when sound associated to jaw clenching is detected. Activation of the signaling unit may alert the user to be aware of the tooth grinding or jaw clenching occurring. In case the user is asleep, the signaling may achieve that the user consciously or unconsciously stops tooth grinding and jaw clenching.

The processing unit preferably is configured to activate the signaling unit such that a first signal is generated when sound associated to tooth grinding is detected and a second signal is generated when sound associated to jaw clenching is detected, wherein the first signal is different from the second signal. By such configuration the user or the practitioner may distinguish between the different bruxism mechanism occurring. Like this, a specific treatment may be applied.

In an embodiment, the earplug body of the bruxism detection device comprises a further external sound sensing transducer or a, e.g. 3-axis, inertial measurement unit (IMU), which can be placed on the head of the user at different locations. The measurements made by such additional sensors, e.g. sound signals and/or displacement information, can be used either as reference measurement or to establish certain correlations or both.

Preferably, the bruxism detection device comprises an exterior sound sensing transducer mounted to the earplug body such that the exterior sound sensing transducer is directed towards an auricle of the user when the earplug body is positioned in the ear canal of the user. Thereby, the processing unit preferably is configured to assess the exterior sound signal received from the exterior sound sensing transducer with respect to the sound signal received from the sound sensing transducer. By evaluating the exterior sound sensing transducer, it can be detected which signals or sounds originate from the inside and which ones from the outside of the ear. Like this, e.g. the exterior sound can be cancelled or reduced, which may be an alternative to occlude the ear canal. Also the sounds generated towards the ear canal can be controlled and adjusted.

In another aspect, the invention, as defined in independent claim 10, relates to a method of configuring a bruxism detection device having an earplug body designed to be positioned in an ear canal of a user, a sound sensing transducer mounted to the earplug body such that the sound sensing transducer is directed towards a tympanic membrane of the user, when the earplug body is positioned in the ear canal of the user, and a processing unit connected to the sound sensing transducer such that a sound signal detected by the sound sensing transducer are receivable by the processing unit. The method comprises the steps of:
- applying a machine learning algorithm to detect sound features associated to tooth grinding;
- applying a machine learning algorithm to detect sound features associated to jaw clenching; and
- configuring the processing unit of the bruxism detection device to evaluate the sound signal received from the sound sensing transducer to detect sound associated to tooth grinding and to detect sound associated to jaw clenching on the basis of the detected sound features.

By means of the method according to the invention, a bruxism detection device and, in particular, a bruxism detection device in accordance with any embodiment of the bruxism detection device according to the invention described above can be provided. Like this, the effect and benefits described above in connection with the bruxism detection device according to the invention and its embodiments described above can be achieved. Moreover, the method according to the invention allows for efficiently manufacturing or providing an appropriate and accurate bruxism detection device.

In the method according to the invention, various machine learning algorithms may be applied. They may involve feature based approaches or approaches based on neural networks. Also, a combination of machine learning algorithms may be used which can increase quality of the reference sounds. Thus, any algorithm or algorithm combination suitable for solving the problem of detecting the sound feature(s) to be associated to tooth grinding or those of jaw clenching from the sound signals sensed by the sound sensing transducer can be used. The machine learning algorithm used for determining the sound features associated to tooth grinding can be the same as or different from the machine learning algorithm used for determining the sound features associated to jaw clenching.

Preferably, applying the machine learning algorithm to detect sound features associated to tooth grinding and/or associated to jaw clenching comprises mounting at least one microphone to a number of users, each of the users intentionally grinding teeth and/or clenching jaw and, for each of the users, feeding parameters of sound signals sensed by the at least one microphone to the machine learning algorithm. The users can be users suffering from bruxism or any other persons. Such method allows for efficiently configuring the processing unit of the bruxism detection device and to achieve a high detection accuracy.

A possible way of determining sound features associated to jaw clenching or tooth grinding is to place the microphone, voice pick up sensor or the like in the ear canal of the user or the user and/or on a different location on the skull of the user or user in order to acquire a reference sound signal and connect the sound sensing transducer(s) to a data acquisition device and capture the required data. In an example, the data acquisition device is connected to a PC via USB in order to store the data and the data was sampled at 6 kHz.

After the data acquisition, the data may need to be processed e.g. by using certain filters. In an example, a least-squares linear-phase FIR low pass filter and a least squares linear-phase FIR high pass filter are used. Depending on the architecture or type of the machine learning algorithm other data processing steps may be needed to be executed, such as aggregating the acquired sound features to patterns.

Preferably, the sound features comprise at least one of wavelength, frequency, speed and amplitude. Such parameters allow for efficiently and accurately detect sounds caused by tooth grinding and/or jaw clenching.

Preferably, the method comprises the steps of aggregating the detected sound features associated to tooth grinding to a tooth grinding reference pattern, and aggregating the detected sound features associated to jaw clenching to a jaw clenching reference pattern. By generating such patterns, and efficient processing and evaluation can be achieved.

Further, configuring the processing unit of the bruxism identification device to evaluate the sound signal received from the sound sensing transducer preferably comprises aggregating the sound features of the sound signal received from the sound sensing transducer to a sound pattern. Such sound pattern may further increase efficiency in processing and evaluation.

In an embodiment the step of configuring the processing unit of the bruxism detection device to evaluate the sound signal received from the sound sensing transducer preferably comprises comparing the sound pattern to the tooth grinding reference pattern and comparing the sound pattern to the jaw clenching reference pattern. Such comparison of the patterns allows for a specifically efficient evaluation.

Preferably, the method comprises a step of configuring the processing unit of the bruxism detection device to activate a signaling unit of the bruxism detection device, when sound associated to tooth grinding is detected and/or when sound associated to jaw clenching is detected.

Thereby, configuring the processing unit of the bruxism detection device to activate a signaling unit preferably comprises activating the signaling unit of the bruxism detection device such that a first signal is generated when sound associated to tooth grinding is detected and a second signal is generated when sound associated to jaw clenching is detected, wherein the first signal is different from the second signal.

The method can further comprise the following optional steps:
- once the matching sound pattern is found generating a digital signal to be transferred and transformed into a sound signal in the form of an alert (graphic, haptic or audible) or a calming sound,
- once the matching sound pattern is found send a digital signal to the storage unit in order to start recording the sound signals for a predetected period of time, the recording may start at an earlier stage as well
- the sound signals generated by the user may be used to continuously adapt the tooth grinding reference pattern and/or the jaw clenching reference pattern.

### Brief Description of the Drawings

The device according to the invention and the method according to the invention are described in more detail hereinbelow by way of exemplary embodiments and with reference to the attached drawings, in which:
- Fig. 1: shows a first embodiment of a bruxism detection device according to the invention comprising an earplug body, a sound sensing transducer and a processing unit;
- Fig. 2: shows a second embodiment of a bruxism detection device according to the invention comprising an earplug body, a sound sensing unit, a sound generating transducer and a processing unit;
- Fig. 3: shows a third embodiment of a bruxism detection device according to the invention showing two ways of transmitting digital signals to a remote processing unit;
- Fig. 4: shows a fourth embodiment of a bruxism detection device according to the invention showing two ways of transmitting digital signals to a remote processing unit; and
- Fig. 5: shows two diagrams of acquired sound signals determining sound features associated to jaw clenching or tooth grinding generated in an embodiment of a method according to the invention.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

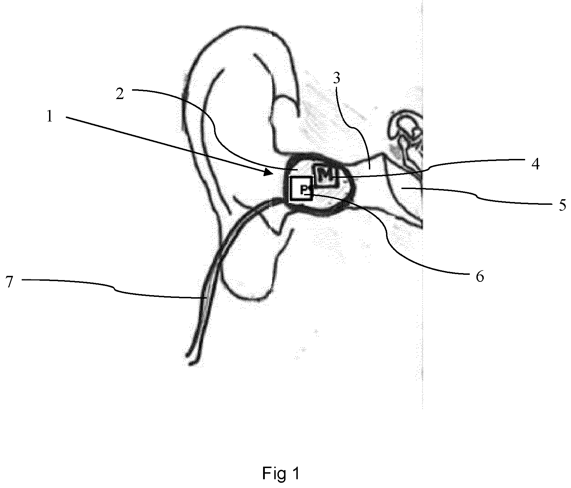

Fig. 1 shows a first embodiment of a bruxism detection device 1 according to the invention. The bruxism detection device 1 comprises an earplug body 2, a microphone 2 as sound sensing transducer and a processing unit 6. The earplug body 2 is designed to be inserted and positioned in an ear canal 3 of a user. It is made of a flexible, gum like material in order to achieve a soundproof fit in the ear canal 3 of the user. In the embodiment of Fig. 1 the microphone 4 as well as the processing unit 6 are integrated into the earplug body 2. Thereby, the sound sensing transducer 4 is mounted in such a way, that it is directed towards a tympanic membrane 5 of the user when the earplug body 2 is positioned in the ear canal 3 of the user.

In other embodiments, the microphone 4 and/or the processing unit 6 may be separate parts of the device being attached to the earplug body 2 via, e.g., wires, attachment structures or the like. The sound generating transducer 8 may also be positioned within the earplug body 2 and having an acoustic canal that it is directed towards a tympanic membrane 5 of the user.

The bruxism detection device of Fig. 1 has a wire 7 extending from the earplug body 2 outside the user's ear. This wire can be connected to an external device being a smart phone, a storage unit or the like. Depending on the exact embodiment, the wire 7 is connected with the processing unit 6 of the device 1.

The processing unit 6 is configured to evaluate a sound signal generated by and received from the microphone 4 to detect sound associated to tooth grinding and sound associated to jaw clenching. In particular, the processing unit 6 is configured to evaluate the sound signal received from the sound sensing transducer by comparing sound features predefined to be associated to tooth grinding to sound features of the sound signal received from the sound sensing transducer. The sound features may comprise at least one of wavelength, frequency, speed and amplitude, or preferably a combination of any of those parameters.

More specifically, in evaluation of the sound signal, the sound features of the sound signal received from the microphone 4 are aggregated to a sound pattern. The sound pattern is compared to a predefined tooth grinding reference pattern and to a predefined jaw clenching reference pattern. If the sound pattern matches the tooth grinding reference pattern and/or to the jaw clenching reference pattern, tooth grinding and/or jaw clenching is detected. Results of the detection are transferred via the wire 7 to the external device.

The tooth grinding reference pattern and the jaw clenching reference pattern are predefined in the processing unit 6 in accordance with the method according to the invention as described in more detail below. In particular, the reference patterns are predefined by means of a machine learning algorithm.

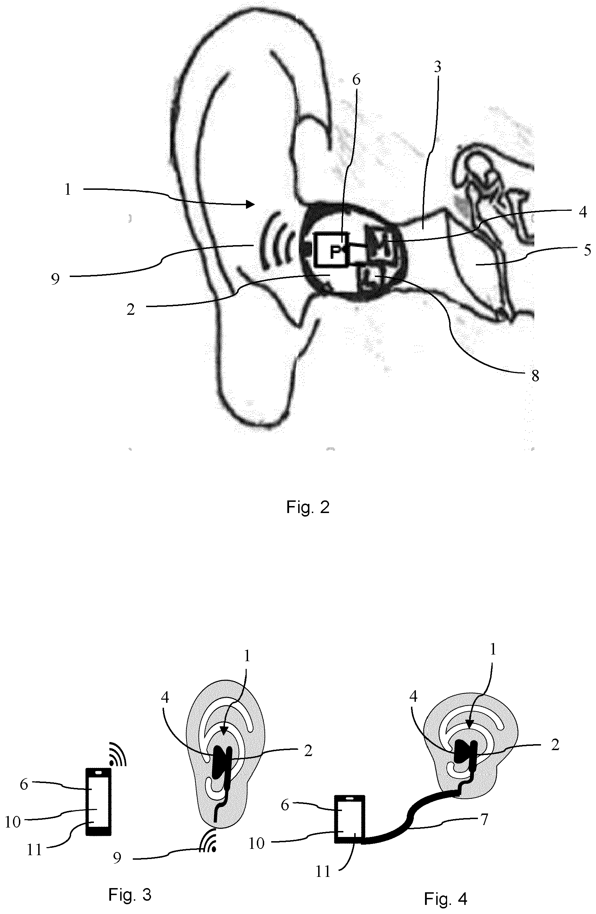

In Fig. 2 a second embodiment of the bruxism detection device 1 according to the invention is shown. This bruxism detection device 1 is similarly embodied as the bruxism detection device 1 of Fig. 1. It basically differs in that a loudspeaker 8 as sound generating transducer is an integral part of the earplug body 2 of the of the bruxism detection device 1. The processing unit 6, the sound sensing transducer 4 and the sound generating transducer 8 are interlinked in the depicted embodiment. The alignment of the sound sensing transducer 4 and the loudspeaker 8 is according to their function towards the tympanic membrane 5 of the user when the earplug body 2 is positioned in the ear canal 3 of the user.

The loudspeaker 8 may in embodiments be a separate part of the device being attached to the earplug body 2 via, e.g., a wire, an attachment structure and the like. Also, in these embodiments the loudspeaker 8 is attached to the earplug body 2 oriented towards the tympanic membrane 5 of the user when the earplug body 2 is positioned in the ear canal 3 of the user. The processing unit 6 is connected to the loudspeaker 8 and is configured to control the loudspeaker 8 to generate a calming sound when sound associated to tooth grinding and/or sound associated to jaw clenching is detected.

Furthermore, in contrast to the wire 7 shown in Fig. 1 the bruxism detection device of Fig. 2 has an antenna 9 for, e.g., Bluetooth communication in order to wirelessly transmit digital data to an external device such as, e.g., an external storage unit.

Fig. 3 and Fig. 4 show two further embodiments of the bruxism detection device according to the invention. In both embodiments, the bruxism detection device 1 comprises an earplug body 2 as described above with an integrated microphone 4 as sound sensing transducer. However, in contrast to the first and second embodiments described above, in the third and fourth embodiments of Figs. 3 and 4 the processing unit 6 is embodied in an external smartphone 10. The smartphone 10 and, thus, the processing unit 6 are connected to the earplug body 2 and, more specifically, to the microphone 4. In particular, in the third embodiment of Fig. 3, the earplug body 2 is equipped with a Bluetooth adapter in communication with the smartphone 10. In the fourth embodiment of Fig. 4, the smartphone 10 and the earplug body 2 are connected by means or a data cable 7 or wire. The smartphone 10 further embodies an optoacoustic signaling unit 11.

In embodiments where the processing unit 6 is an external device or embodied by an external device, the wire can be connected to the sound sensing transducer 4 and the processing unit 6. The number of wires is not limited to one wire only - for instance a second wire can be connected to the processing unit being an external device and the sound generating transducer 4 being a part of the earplug body 2 (integrated or attached).

All embodiments of bruxism detection devices 1 described above in connection with the Figs. are configured in accordance with an embodiment of a method according to the invention. In particular such configuration comprises the steps of:
(i) applying a machine learning algorithm to detect sound features associated to tooth grinding;
(ii) applying another or the same machine learning algorithm to detect sound features associated to jaw clenching, and
(iii) configuring the processing unit of the bruxism detection device to evaluate the sound signal received from the sound sensing transducer to detect sound associated to tooth grinding and to detect sound associated to jaw clenching on the basis of the detected sound features.

In step (i) plural bone conducting microphones and voice pick up sensors are mounted at different appropriate locations, particularly locations at the head, to a number of users one after the other. Each of the users intentionally grinds teeth. For each of the users, parameters of sound signals sensed by the bone conducting microphones and voice pick up sensors are fed to the machine learning algorithm. The sound features detected like this are then aggregated to a tooth grinding reference pattern, which is made available to or predefined in the processing unit 6.

Similarly, in step (ii) plural bone conducting microphones and voice pick up sensors are mounted at different locations, particularly locations at the head, to a number of users one after the other. Each of the users intentionally clenches teeth. For each of the users, parameters of sound signals sensed by the bone conducting microphones and voice pick up sensors are fed to the machine learning algorithm. The sound features detected like this are then aggregated to a jaw clenching reference pattern, which is made available to or predefined in the processing unit 6.

In step (iii) the processing unit 6 is configured to aggregate the sound features of the sound signal received from the microphone 4 to a sound pattern. Further, the processing unit 6 is configured to compare the sound pattern to the tooth grinding reference pattern and to compare the sound pattern to the jaw clenching reference pattern. In the embodiments of Fig. 3 and of Fig. 4, the processing unit 6 is further configured to activate the signaling unit 11 in the smartphone to provide an acoustic and optical signal to the user, when sound associated to tooth grinding is detected and/or when sound associated to jaw clenching is detected. In particular, the processing unit 6 is configured to provide different signals via the signaling unit when sound associated to tooth grinding is detected, when sound associated to jaw clenching is detected or when sound associated to both is detected.

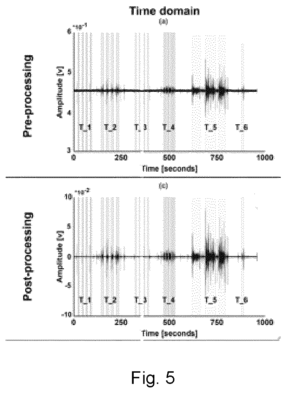

Fig 5 shows two diagrams of sound features (i.e. at least one of wavelength, frequency, speed and amplitude) detected by a test setup in order to obtain sound signals dedicated to be sound features associated to jaw clenching or tooth grinding according to the method of configuring a bruxism detection device 1. The data shown in the diagrams are based on measurements made by six bone conducting microphones placed on the skull of a user and two voice pick up sensor placed in the ear canal of a user. The user conducted 5 different mandibular activities while recording the measurements of the sound sensing transducers (T1-T6): T 1 (jaw clenching), T 2 (tooth grinding), T 3 (jaw clenching), T 4 (reading), T 5 (eating), and T 6 (drinking). The shaded areas in the diagram of Fig. 4 represent the periods where the user/patient was active.

The top diagram of Fig. 5 illustrates the time domain and frequency domain representation of the voice pick up sensor (placed in the left ear canal) output before processing. The voice pick up sensors' output is oscillating around 0.45 volts. T 5 has the widest oscillation range of [0.38 0.53] compared to [0.41 0.48], [0.43 0.46], and [0.42 0.46] for T 2, T 4, and T 6, respectively. The clenching tasks T 1 and T 3 is not observed.

The second diagram of Fig. 5 illustrates the voice pick up sensors' output after processing the data. Similarly, T 5 has the widest peak-to-peak amplitude between [-0.06 0.08] compared to [-0.03 0.02], [-0.01 0.01], and [-0.02 0.01] for T 2, T 4, and T 6, respectively. Also, the clenching tasks, T 1 and T 3, were not observed in the time domain.

This shows an example the measurable difference - here amplitude - between the sound features associated to jaw clenching and the sound features associated to tooth grinding and how such parameters can be acquired.

## Claims

1. A bruxism detection device (1) comprising
an earplug body (2) designed to be positioned in an ear canal (3) of a user;
a sound sensing transducer (4) mounted to the earplug body (2) such that the sound sensing transducer (4) is directed towards a tympanic membrane (5) of the user when the earplug body (2) is positioned in the ear canal (3) of the user; and
a processing unit (6) connected to the sound sensing transducer (4) such that a sound signal detected by the sound sensing transducer (4) is receivable by the processing unit (6),
the processing unit (6) is configured to evaluate the sound signal received from the sound sensing transducer (4) to detect sound associated to tooth grinding,
**characterised in that**
the processing unit (6) is further configured to evaluate the sound signal received from the sound sensing transducer (4) to detect sound associated to jaw clenching.

2. The bruxism detection device (1) of claim 1, wherein the processing unit (6) is configured to evaluate the sound signal received from the sound sensing transducer (4) by means of a machine learning algorithm.

3. The bruxism detection device (1) of claim 1 or 2, wherein the processing unit (6) is configured to evaluate the sound signal received from the sound sensing transducer (4) by comparing sound features predefined to be associated to tooth grinding to sound features of the sound signal received from the sound sensing transducer (4).

4. The bruxism detection device (1) of any one of the preceding claims, wherein the processing unit (6) is configured to evaluate the sound signal received from the sound sensing transducer (4) by comparing sound features predefined to be associated to jaw clenching to sound features of the sound signal received from the sound sensing transducer (4).

5. The bruxism detection device (1) of any one of the preceding claims, wherein the earplug body (2) is designed to occlude the ear canal (3) when being positioned in the ear canal (3).

6. The bruxism detection device (1) of any one of the preceding claims, comprising a signaling unit (11) adapted to generate a signal recognizable by the user wherein the processing unit (6) preferably is configured to activate the signaling unit (11), when sound associated to tooth grinding is detected and/or when sound associated to jaw clenching is detected.

7. The bruxism detection device (1) of claim 6, wherein the processing unit (6) is configured to activate the signaling unit (11) such that a first signal is generated when sound associated to tooth grinding is detected and a second signal is generated when sound associated to jaw clenching is detected, wherein the first signal is different from the second signal.

8. The bruxism detection device (1) of any one of the preceding claims, comprising a sound generating transducer (8) mounted to the earplug body (2) such that the sound generating transducer (8) is directed towards the tympanic membrane (5) of the user, when the earplug body (2) is positioned in the ear canal (3) of the user, wherein the processing unit (6) preferably is connected to the sound generating transducer (8) and wherein the processing unit (6) preferably is configured to control the sound generating transducer (8) to generate a calming sound when sound associated to tooth grinding and/or sound associated to jaw clenching is detected.

9. The bruxism detection device (1) of any one of the preceding claims, comprising an exterior sound sensing transducer mounted to the earplug body (2) such that the exterior sound sensing transducer is directed towards an auricle of the user when the earplug body (2) is positioned in the ear canal (3) of the user, wherein the processing unit (6) preferably is configured to assess the exterior sound signal received from the exterior sound sensing transducer with respect to the sound signal received from the sound sensing transducer (4).

10. A method of configuring a bruxism detection device (1) having an earplug body (2) designed to be positioned in an ear canal (3) of a user, a sound sensing transducer (4) mounted to the earplug body (2) such that the sound sensing transducer (4) is directed towards a tympanic membrane (5) of the user, when the earplug body (2) is positioned in the ear canal (3) of the user, and a processing unit (6) connected to the sound sensing transducer (4) such that a sound signal detected by the sound sensing transducer (4) are receivable by the processing unit (6), the method comprising
applying a machine learning algorithm to detect sound features associated to tooth grinding wherein such sound features preferably comprise at least one of wavelength, frequency, speed and amplitude,
applying a machine learning algorithm to detect sound features associated to jaw clenching wherein such sound features preferably comprise at least one of wavelength, frequency, speed and amplitude, and
configuring the processing unit (6) of the bruxism detection device (1) to evaluate the sound signal received from the sound sensing transducer (4) to detect sound associated to tooth grinding and to detect sound associated to jaw clenching on the basis of the detected sound features.

11. The method of claim 10, wherein applying the machine learning algorithm
to detect sound features associated to tooth grinding comprises mounting at least one microphone to a number of users, each of the users intentionally grinding teeth and, for each of the users, feeding parameters of sound signals sensed by the at least one microphone to the machine learning algorithm, and or
to detect sound features associated to jaw clenching comprises mounting at least one microphone to a number of users, each of the users intentionally clenching teeth and, for each of the users, feeding parameters of sound signals sensed by the at least one microphone to the machine learning algorithm.

12. The method of claim 10 or 11, comprising
aggregating the detected sound features associated to tooth grinding to a tooth grinding reference pattern, and
aggregating the detected sound features associated to jaw clenching to a jaw clenching reference pattern.

13. The method of any one of claims 10 to 12, wherein configuring the processing unit (6) of the bruxism detection device (1) to evaluate the sound signal received from the sound sensing transducer (4) comprises aggregating the sound features of the sound signal received from the sound sensing transducer (4) to a sound pattern.

14. The method of claims 12 and 13, wherein configuring the processing unit (6) of the bruxism detection device (1) to evaluate the sound signal received from the sound sensing transducer (4) comprises comparing the sound pattern to the tooth grinding reference pattern and comparing the sound pattern to the jaw clenching reference pattern.

15. The method of any one of claims 10 to 14, comprising a step of configuring the processing unit (6) of the bruxism detection device (1) to activate a signaling unit (11) of the bruxism detection device (1), when sound associated to tooth grinding is detected and/or when sound associated to jaw clenching is detected, wherein configuring the processing unit (6) of the bruxism detection device (1) to activate a signaling unit (11) preferably comprises activating the signaling unit (11) of the bruxism detection device (1) such that a first signal is generated when sound associated to tooth grinding is detected and a second signal is generated when sound associated to jaw clenching is detected, wherein the first signal is different from the second signal.

## Patentansprüche

1. Eine Bruxismus-Erkennungsvorrichtung (1) aufweisend
einen Ohrstöpselkörper (2) zur Positionierung im Gehörgang (3) eines Benutzers;
einen schallempfindlichen Wandler (4), der am Ohrstöpselkörper (2) so angebracht ist, dass er auf das Trommelfell (5) des Benutzers gerichtet ist, wenn sich der Ohrstöpselkörper (2) im Gehörgang (3) des Benutzers befindet; und
eine Verarbeitungseinheit (6), die mit dem schallempfindlichen Wandler (4) verbunden ist, sodass ein vom schallempfindlichen Wandler (4) erfasstes Schallsignal von der Verarbeitungseinheit (6) empfangen werden kann,
die Verarbeitungseinheit (6) ist so konfiguriert, dass sie das vom schallempfindlichen Wandler (4) empfangene Schallsignal auswertet, um Geräusche im Zusammenhang mit Zähneknirschen zu erkennen, **dadurch gekennzeichnet, dass**
die Verarbeitungseinheit (6) ferner so konfiguriert ist, dass sie das vom schallempfindlichen Wandler (4) empfangene Schallsignal auswertet, um Geräusche im Zusammenhang mit Kieferpressen zu erkennen.

2. Die Bruxismus-Erkennungsvorrichtung (1) nach Anspruch 1, wobei die Verarbeitungseinheit (6) so konfiguriert ist, dass sie das vom schallempfindlichen Wandler (4) empfangene Schallsignal mittels eines Algorithmus für maschinelles Lernen auswertet.

3. Die Bruxismus-Erkennungsvorrichtung (1) nach Anspruch 1 oder 2, wobei die Verarbeitungseinheit (6) so konfiguriert ist, dass sie das vom schallempfindlichen Wandler (4) empfangene Schallsignal auswertet, indem sie vordefinierte, mit Zähneknirschen assoziierte Schallmerkmale mit den Schallmerkmalen des vom schallempfindlichen Wandler (4) empfangenen Schallsignals vergleicht.

4. Die Bruxismus-Erkennungsvorrichtung (1) nach einem der vorhergehenden Ansprüche, wobei die Verarbeitungseinheit (6) so konfiguriert ist, dass sie das vom schallempfindlichen Wandler (4) empfangene Schallsignal auswertet, indem sie vordefinierte, mit Kieferpressen assoziierte Schallmerkmale mit den Schallmerkmalen des vom schallempfindlichen Wandler (4) empfangenen Schallsignals vergleicht.

5. Die Bruxismus-Erkennungsvorrichtung (1) nach einem der vorhergehenden Ansprüche, wobei der Ohrstöpselkörper (2) so gestaltet ist, dass er den Gehörgang (3) verschließt, wenn er in diesen eingesetzt wird.

6. Die Bruxismus-Erkennungsvorrichtung (1) nach einem der vorhergehenden Ansprüche, umfassend eine Signaleinheit (11) zur Erzeugung eines für den Benutzer erkennbaren Signals, wobei die Verarbeitungseinheit (6) vorzugsweise so konfiguriert ist, dass sie die Signaleinheit (11) aktiviert, wenn Geräusche im Zusammenhang mit Zähneknirschen erkannt werden und/oder wenn Geräusche im Zusammenhang mit Kieferpressen erkannt werden.

7. Die Bruxismus-Erkennungsvorrichtung (1) nach Anspruch 6, wobei die Verarbeitungseinheit (6) so konfiguriert ist, dass sie die Signaleinheit (11) so aktiviert, dass beim Erkennen von Geräuschen im Zusammenhang mit Zähneknirschen ein erstes Signal erzeugt wird und beim Erkennen von Geräuschen im Zusammenhang mit Kieferpressen ein zweites Signal erzeugt wird, wobei sich das erste Signal vom zweiten Signal unterscheidet.

8. Die Bruxismus-Erkennungsvorrichtung (1) nach einem der vorhergehenden Ansprüche, umfassend einen am Ohrstöpselkörper (2) angebrachten schallerzeugenden Wandler (8), der auf das Trommelfell (5) des Benutzers gerichtet ist, wenn sich der Ohrstöpselkörper (2) im Gehörgang (3) des Benutzers befindet, wobei die Verarbeitungseinheit (6) vorzugsweise mit dem schallerzeugenden Wandler (8) verbunden ist und vorzugsweise so konfiguriert ist, dass sie den schallerzeugenden Wandler (8) steuert, um ein beruhigendes Geräusch zu erzeugen, wenn Geräusche im Zusammenhang mit Zähneknirschen und/oder Geräusche im Zusammenhang mit Kieferpressen erkannt werden.

9. Die Bruxismus-Erkennungsvorrichtung (1) nach einem der vorhergehenden Ansprüche, umfassend einen externen schallempfindlichen Wandler, der am Ohrstöpselkörper (2) so angebracht ist, dass der externe schallempfindliche Wandler auf die Ohrmuschel des Benutzers gerichtet ist, wenn sich der Ohrstöpselkörper (2) im Gehörgang (3) des Benutzers befindet, wobei die Verarbeitungseinheit (6) vorzugsweise so konfiguriert ist, dass sie das vom externen schallempfindlichen Wandler empfangene externe Schallsignal im Verhältnis zum vom schallempfindlichen Wandler (4) empfangenen Schallsignal auswertet.

10. Verfahren zur Konfiguration eines Bruxismus-Erkennungsgeräts (1) mit einem Ohrstöpselkörper (2) zur Positionierung im Gehörgang (3) eines Benutzers, einem am Ohrstöpselkörper (2) angebrachten schallempfindlichen Wandler (4), der auf das Trommelfell (5) des Benutzers gerichtet ist, wenn sich der Ohrstöpselkörper (2) im Gehörgang (3) des Benutzers befindet, und einer mit dem schallempfindlichen Wandler (4) verbundenen Verarbeitungseinheit (6), die ein vom schallempfindlichen Wandler (4) erfasstes Schallsignal empfängt, wobei das Verfahren umfasst:
Anwenden eines Algorithmus für maschinelles Lernen zur Erkennung von Schallmerkmalen, die mit Zähneknirschen in Verbindung stehen, wobei diese Schallmerkmale vorzugsweise zumindest Wellenlänge, Frequenz, Geschwindigkeit oder Amplitude aufweisen;
Anwenden eines Algorithmus für maschinelles Lernen zur Erkennung von Schallmerkmalen, die mit Kieferpressen in Verbindung stehen, wobei diese Schallmerkmale vorzugsweise zumindest Wellenlänge, Frequenz, Geschwindigkeit oder Amplitude aufweisen; und
Konfigurieren der Verarbeitungseinheit (6) des Bruxismus-Erkennungsgeräts. (1) das vom schallempfindlichen Wandler (4) empfangene Schallsignal auszuwerten, um auf der Grundlage der erfassten Schallmerkmale Geräusche zu erkennen, die mit Kieferpressen verbunden sind.

11. Verfahren nach Anspruch 10, wobei der Algorithmus für maschinelles Lernen angewendet wird
zur Erkennung von mit Zähneknirschen verbundenen Geräuschmerkmalen das Anbringen zumindest eines Mikrofons an mehreren Benutzern umfasst, wobei jeder Benutzer absichtlich mit den Zähnen knirscht und für jeden Benutzer die Parameter der von dem mindestens einen Mikrofon erfassten Schallsignale dem Algorithmus für maschinelles Lernen zugeführt werden, und/oder
zur Erkennung von mit Kieferpressen verbundenen Geräuschmerkmalen das Anbringen mindestens eines Mikrofons an mehreren Benutzern umfasst, wobei jeder Benutzer absichtlich mit den Zähnen presst und für jeden Benutzer die Parameter der von dem mindestens einen Mikrofon erfassten Schallsignale dem Algorithmus für maschinelles Lernen zugeführt werden.

12. Verfahren nach Anspruch 10 oder 11, umfassend
das Zusammenfassen der erfassten, mit Zähneknirschen verbundenen Schallmerkmale zu einem Referenzmuster für Zähneknirschen und
das Zusammenfassen der erfassten, mit Kieferpressen verbundenen Schallmerkmale zu einem Referenzmuster für Kieferpressen.

13. Verfahren nach einem der Ansprüche 10 bis 12, wobei die Verarbeitungseinheit (6) der Bruxismus-Erkennungsvorrichtung (1) zur Auswertung des vom schallempfindlichen Wandler (4) empfangenen Schallsignals das Zusammenfassen der Schallmerkmale des vom schallempfindlichen Wandler (4) empfangenen Schallsignals zu einem Schallmuster umfasst.

14. Verfahren nach den Ansprüchen 12 und 13, wobei die Verarbeitungseinheit (6) der Bruxismus-Erkennungsvorrichtung (1) zur Auswertung des vom schallempfindlichen Wandler (4) empfangenen Schallsignals das Vergleichen des Schallmusters mit dem Referenzmuster für Zähneknirschen und das Vergleichen des Schallmusters mit dem Referenzmuster für Kieferpressen umfasst.

15. Verfahren nach einem der Ansprüche 10 bis 14, umfassend einen Schritt des Konfigurierens der Verarbeitungseinheit (6) der Bruxismus-Erkennungsvorrichtung (1) zur Aktivierung einer Signaleinheit (11) der Bruxismus-Erkennungsvorrichtung (1), wenn ein Geräusch im Zusammenhang mit Zähneknirschen erkannt wird und/oder ein Geräusch im Zusammenhang mit Kieferpressen erkannt wird, wobei das Konfigurieren der Verarbeitungseinheit (6) der Bruxismus-Erkennungsvorrichtung (1) zur Aktivierung einer Signaleinheit (11) vorzugsweise das Aktivieren der Signaleinheit (11) der Bruxismus-Erkennungsvorrichtung (1) umfasst, sodass ein erstes Signal erzeugt wird, wenn ein Geräusch im Zusammenhang mit Zähneknirschen erkannt wird, und ein zweites Signal erzeugt wird, wenn ein Geräusch im Zusammenhang mit Kieferpressen erkannt wird, wobei sich das erste Signal vom zweiten Signal unterscheidet.

## Revendications

1. Dispositif de détection du bruxisme (1) comprenant
un corps de bouchon d'oreille (2) conçu pour être positionné dans le conduit auditif (3) de l'utilisateur ;
un transducteur de détection sonore (4) monté sur le corps du bouchon d'oreille (2) de manière à être dirigé vers la membrane tympanique (5) de l'utilisateur lorsque le corps du bouchon d'oreille (2) est positionné dans son conduit auditif (3) ; et
une unité de traitement (6) connectée au transducteur de détection sonore (4) de manière à recevoir par l'unité de traitement (6) tout signal sonore détecté par le transducteur de détection sonore (4)
l'unité de traitement (6) est configurée pour analyser le signal sonore reçu du transducteur de détection sonore (4) afin de détecter les bruits associés au grincement des dents, **caractérisé en ce que**
l'unité de traitement (6) est également configurée pour analyser le signal sonore reçu du transducteur de détection sonore (4) afin de détecter les bruits associés au serrement des mâchoires.

2. Dispositif de détection du bruxisme (1) selon la revendication 1, dans lequel l'unité de traitement (6) est configurée pour évaluer le signal sonore reçu du transducteur de détection sonore (4) au moyen d'un algorithme d'apprentissage automatique.

3. Dispositif de détection du bruxisme (1) selon la revendication 1 ou 2, dans lequel l'unité de traitement (6) est configurée pour évaluer le signal sonore reçu du transducteur de détection sonore (4) en comparant des caractéristiques sonores prédéfinies, associées au grincement des dents, aux caractéristiques sonores du signal reçu du transducteur de détection sonore (4).

4. Dispositif de détection du bruxisme (1) selon l'une quelconque des revendications précédentes, dans lequel l'unité de traitement (6) est configurée pour évaluer le signal sonore reçu du transducteur de détection sonore (4) en comparant des caractéristiques sonores prédéfinies, associées au serrement des mâchoires, aux caractéristiques sonores du signal reçu du transducteur de détection sonore (4).

5. Dispositif de détection du bruxisme (1) selon l'une quelconque des revendications précédentes, dans lequel le corps du bouchon d'oreille (2) est conçu pour obstruer le conduit auditif (3) lorsqu'il est positionné dans celui-ci.

6. Dispositif de détection du bruxisme (1) selon l'une quelconque des revendications précédentes, comprenant une unité de signalisation (11) adaptée pour générer un signal reconnaissable par l'utilisateur, l'unité de traitement (6) étant de préférence configurée pour activer l'unité de signalisation (11) lors de la détection d'un bruit associé au grincement des dents et/ou lors de la détection d'un bruit associé au serrement des mâchoires.

7. Dispositif de détection du bruxisme (1) selon la revendication 6, dans lequel l'unité de traitement (6) est configurée pour activer l'unité de signalisation (11) de sorte qu'un premier signal soit généré lors de la détection d'un bruit associé au grincement des dents et un second signal lors de la détection d'un bruit associé au serrement des mâchoires, le premier signal étant différent du second.

8. Le dispositif de détection du bruxisme (1) selon l'une quelconque des revendications précédentes, comprenant un transducteur de génération de son (8) monté sur le corps du bouchon d'oreille (2) de sorte que le transducteur de génération de son (8) soit dirigé vers la membrane tympanique (5) de l'utilisateur lorsque le corps du bouchon d'oreille (2) est positionné dans le conduit auditif (3) de l'utilisateur, l'unité de traitement (6) étant de préférence connectée au transducteur de génération de son (8) et configurée de préférence pour commander ce dernier afin de générer un son apaisant lorsqu'un bruit associé au grincement des dents et/ou au serrement des mâchoires est détecté.

9. Le dispositif de détection du bruxisme (1) selon l'une quelconque des revendications précédentes, comprenant un transducteur de détection sonore externe monté sur le corps du bouchon d'oreille (2) de sorte que le transducteur de détection sonore externe soit dirigé vers l'oreille de l'utilisateur lorsque le corps du bouchon d'oreille (2) est positionné dans le conduit auditif (3) de l'utilisateur, dans lequel l'unité de traitement (6) est de préférence configurée pour évaluer le signal sonore externe reçu du transducteur de détection sonore externe par rapport au signal sonore reçu du transducteur de détection sonore (4).

10. Procédé de configuration d'un dispositif de détection du bruxisme (1) comprenant un corps de bouchon d'oreille (2) conçu pour être positionné dans le conduit auditif (3) de l'utilisateur, un transducteur de détection sonore (4) monté sur le corps de bouchon d'oreille (2) de manière à être dirigé vers la membrane tympanique (5) de l'utilisateur lorsque l'embout (2) est positionné dans le conduit auditif (3), et une unité de traitement (6) connectée au transducteur de détection sonore (4) de manière à recevoir par l'unité de traitement (6) les signaux sonores détectés par ce dernier. Le procédé comprend les étapes suivantes :
application d'un algorithme d'apprentissage automatique pour détecter les caractéristiques sonores associées au grincement des dents, ces caractéristiques comprenant de préférence au moins une des valeurs suivantes : longueur d'onde, fréquence, vitesse et amplitude ;
application d'un algorithme d'apprentissage automatique pour détecter les caractéristiques sonores associées au serrement des mâchoires, ces caractéristiques comprenant de préférence au moins une des valeurs suivantes : longueur d'onde, fréquence, vitesse et amplitude ;
configuration de l'unité de traitement (6) du dispositif de détection du bruxisme (1) pour évaluer le signal sonore reçu du transducteur de détection sonore (4) pour détecter le son associé au grincement des dents et pour détecter le son associé au serrement de la mâchoire sur la base des caractéristiques sonores détectées.

11. Procédé selon la revendication 10, dans lequel l'application de l'algorithme d'apprentissage automatique
pour détecter les caractéristiques sonores associées au grincement des dents comprend la fixation d'au moins un microphone sur plusieurs utilisateurs, chacun d'eux grincant intentionnellement des dents et, pour chacun d'eux, la transmission des paramètres des signaux sonores captés par le ou les microphones à l'algorithme d'apprentissage automatique, et/ou
pour détecter les caractéristiques sonores associées au serrement des mâchoires comprend la fixation d'au moins un microphone sur plusieurs utilisateurs, chacun d'eux serrant intentionnellement les dents et, pour chacun d'eux, la transmission des paramètres des signaux sonores captés par le ou les microphones à l'algorithme d'apprentissage automatique.

12. Procédé selon la revendication 10 ou 11, comprenant :
l'agrégation des caractéristiques sonores détectées associées au grincement des dents en un modèle de référence de grincement des dents, et
l'agrégation des caractéristiques sonores détectées associées au serrement de la mâchoire en un modèle de référence de serrement de la mâchoire.

13. Procédé selon l'une quelconque des revendications 10 à 12, dans lequel la configuration de l'unité de traitement (6) du dispositif de détection du bruxisme (1) pour évaluer le signal sonore reçu du transducteur de détection sonore (4) comprend l'agrégation des caractéristiques sonores du signal reçu du transducteur de détection sonore (4) en un modèle sonore.

14. Procédé selon les revendications 12 et 13, dans lequel la configuration de l'unité de traitement (6) du dispositif de détection du bruxisme (1) pour évaluer le signal sonore reçu du transducteur de détection sonore (4) comprend la comparaison du modèle sonore au modèle de référence du grincement des dents et la comparaison du modèle sonore au modèle de référence du serrement des mâchoires.

15. Procédé selon l'une quelconque des revendications 10 à 14, comprenant une étape de configuration de l'unité de traitement (6) du dispositif de détection du bruxisme (1) pour activer une unité de signalisation (11) du dispositif de détection du bruxisme (1), lorsqu'un son associé au grincement des dents est détecté et/ou lorsqu'un son associé au serrement des mâchoires est détecté, dans lequel la configuration de l'unité de traitement (6) du dispositif de détection du bruxisme (1) pour activer une unité de signalisation (11) comprend de préférence l'activation de l'unité de signalisation (11) du dispositif de détection du bruxisme (1) de sorte qu'un premier signal soit généré lorsque le son associé au grincement des dents est détecté et un deuxième signal est généré lorsque le son associé au serrement des mâchoires est détecté, le premier signal étant différent du deuxième signal.
